# EUROPEAN PATENT APPLICATION

(11) **EP 2 719 771 A1**
(43) Date of publication of application: **16.04.2014**
(21) Application number: 12382393.2
(22) Date of filing: 11.10.2012
(51) Int. Cl.: C12Q 1/68

(54) **Marker for assessing the risk of developing acute kidney injury**

(71) Applicant: Ciber de Enfermedades Respiratorias (CIBERES), 07110 Bunyola, Palma de Mallorca (ES); Fundación Para la Investigación Biomédica del Hospital Universitario de Getafe, 28905 Getafe (ES)
(72) Inventor: Cardinal Fernández, Pablo, 28901 Getafe (ES); Ferruelo, Antonio, 28523 Rivas-Vaciamadrid (ES); Lorente, José A., 28035 Madrid (ES)
(74) Representative: ZBM Patents - Zea, Barlocci & Markvardsen

(57) **Abstract**

It is provided an *in vitro* method to determine the risk to develop acute kidney injury (AKI) in a subject, the method comprising the step of detecting the presence of a CC polymorphism at position +936 of the human vascular endothelial growth factor (VEGF) from an isolated sample of the subject, wherein the presence of said polymorphism indicates the risk to develop AKI. With the method of the invention, a new tool to be used in the clinic is made available, allowing the prognosis of a serious life-threatening complication. Additionally, the use of means for detecting the polymorphism are also claimed.

## Description

The present invention provides a method and a marker for determining the risk to develop acute kidney injury in a subject. This invention has potential applications in the area of clinical prognosis.

### BACKGROUND ART

Acute kidney injury (AKI), also known as acute renal failure (ARF), is a very serious complication of critical illness caused by the loss of kidney function. It is said to occur from 1 % to 35% of critically ill patients. Mortality in the patient population suffering from AKI is very high, ranging from 28% to 90%.

Certain patient populations, such as the elderly, diabetics, and patients with renal disease have an especially high risk of developing the disease. AKI can manifest itself in many varied clinical settings, such as cardiac surgery, organ transplants, aortic aneurism repair or exposure to chemicals. However, one of the main leading causes of AKI in the intensive care unit (ICU) is sepsis and septic shock. It is known that septic AKI is linked to a higher mortality rate than non-septic AKI. Due to this very high mortality rate, to its impact on the health system and to the fact that septic AKI is becoming increasingly important, a search for novel ways to improve prognosis of AKI is warranted and, in particular, in sepsis patients.

In the last years, many efforts have been made in elucidating the genetic basis of AKI. In this regard, there is an increasing number of studies linking certain markers and polymorphisms to the likelihood of disease development.

The markers that have been associated with the development of AKI are diverse. In a recent disclosure, the sensitivity, specificity, and prognostic ability of nine urinary biomarkers for AKI and AKI-associated mortality was evaluated (the causes of AKI were diverse, ranging from postcardiac surgery and nephrotoxin administration, to sepsis). It was found that the nine biomarkers analyzed KIM-1, NGAL, IL-18, HGF, Cystatin C, NAG, VEGF and IP-10 and total protein) were able to differentiate between AKI and non-AKI groups of patients (Vaidya VS., et. al. "Urinary biomarkers for sensitive and specific detection of acute kidney injury in humans" Clin. Transl. Sci. 2008, vol 1., pp. 200-2008). However, although these types of studies are relevant, they do not point to which key genetic differences are the main drivers in the progression of sepsis-related AKI.

In an attempt to find out which genetic factors explain the differential susceptibility of patients to develop AKI, several groups have tested the association of certain polymorphisms to this disease. One of the protein families whose polymorphisms have been associated to AKI (and its outcome) are the cytokines. A prospective study was disclosed in which the association of single nucleotide polymorphisms (SNPs) in the promoter regions of TNF-alpha and IL-10 to mortality in AKI patients was tested (Jaber, BL. et al., "Cytokine gene promoter polymorphisms and mortality in acute renal failure", Cytokine 2004, vol. 25, pp. 212-219). It was demonstrated that mortality in a cohort of AKI patients, in which 64% had sepsis, was significantly higher in AKI patients with the -308 A polymorphism located in the promoter of the TNF-alpha gene, and the -1082 A polymorphism located in the promoter of the IL-10 gene.

In a very recent disclosure, a large-scale genotyping effort was conducted on a fairly large cohort of septic shock patients (totalling 1264) in order to discover genetic factors that would significantly associate to the development of AKI. It was reasoned that, because the pathophysiology of AKI can be different depending on the etiology, only patients with a common risk factor (i.e. sepsis or septic shock) would be considered in the study. It was found that two single nucleotide polymorphisms in the BCL2 gene and a single nucleotide polymorphism in the SERPINA4 gene were associated with decreased risk of developing acute kidney injury in patients with sepsis (Frank, A. "BCL2 genetic variants are associated with acute kidney injury in septic shock", Crit. Care Med. 2012, vol. 40, pp. 2116-2123). This document highlights the presence of polymorphisms that can be successfully used to prospectively determine the risk of developing AKI in patients suffering from septic shock.

Unfortunately, as pointed out in a recent disclosure reviewing the state of the art of genetic risk factors associated to AKI (Lu J.C.T. "Searching for genes that matter in acute kidney injury: A systematic review", Clin. J. Am. Soc. Nephrol. 2009, vol. 4, pp. 1020-1030), many confounding variables play a key role in these studies, and new confirmatory evaluations, using more rigorous epidemiologic techniques together with further polymorphisms and their uses are still needed before the genetic basis of sepsis-related AKI is fully unveiled.

In view of the above, there still is an urgent need in the field of prognosis to discover new tools in order to define the most predictive models for acute kidney injury.

### SUMMARY OF THE INVENTION

Inventors have found that a polymorphism located in the gene coding for the vascular endothelial growth factor (VEGF) can be used to assess the predisposition of a patient suffering from sepsis or septic shock to develop acute kidney injury.

In particular, inventors have surprisingly found in a multivariate statistical analysis that +936 CC genotype was significantly associated with development of acute kidney injury.

The SNP +936 of VEGF has previously been linked to the Acute Respiratory Distress Syndrome (ARDS), which is one of the most extreme forms of acute lung injury. However, in ARDS, it was seen that individuals with the CT (hegerozygous) and TT (homozygous) genotypes are more susceptible to ARDS than individuals who are homozygous CC carriers. That is, in this lung disease, it was found that the CC genotype is protective, which is exactly opposite to the present invention (Medford, A.R.L. et. al. "Vascular endothelial growth factor gene polymorphism and acute respiratory distress syndrome" Thorax 2005, Vvol. 60, pp. 244-248). Surprisingly, the present inventors have found the CC genotype is the one that causes a risk to develop AKI in patients. The invention disclosed herewith, therefore, provides with a new means to predict the risk to develop AKI.

Thus, a first aspect of the present invention is an *in vitro* method to determine the risk to develop acute kidney injury in a subject, the method comprising the step of detecting the presence of a CC polymorphism at position +936 of the human vascular endothelial growth factor (VEGF) from an isolated sample of the subject, wherein the presence of said polymorphism indicates the risk to develop acute kidney injury.

Contrary to some of the references found in the prior art, such as Jaber et. al. (see above), the invention can be used not to determine the final outcome of AKI patients, but the genetic predisposition of the patient to develop the disease. This will allow to determine the risk to develop AKI in patients before they undergo any type of clinical intervention that can trigger the disease, such as cardiac surgery, organ transplant, and others.

The method of the present invention is a new predictive tool which translates into clinical advantages, such as the closer monitoring of patients who have been found to be carriers of the polymorphism that associates with the risk, and to devise new treatment protocols for this patient population.

Thus, in a second aspect, the present invention provides a method of deciding or recommending whether to initiate a medical regimen of a subject with a risk of developing AKI, which method comprises determining, *in vitro,* the presence of CC polymorphism at position +936 of VEGF in an isolated sample as defined in the method of the first aspect of the invention, wherein: i) if the subject has the risk of developing AKI, then the initiation of the medical regimen is recommended, and ii) if the subject has not the risk of developing AKI, the follow-up is performed optionally in consideration of the result of an examination of the patient by a physician.

A third aspect of the present invention is the use of means for detecting the presence of a CC polymorphism at position +936 of the human vascular endothelial growth factor (VEGF) from an isolated sample in the method of the first aspect of the invention.

A fourth aspect of the present invention is the use of a CC polymorphism at position +936 of the human vascular endothelial growth factor (VEGF) as marker for determining the risk for developing of acute kidney injury

### BRIEF DESCRIPTION OF THE DRAWINGS

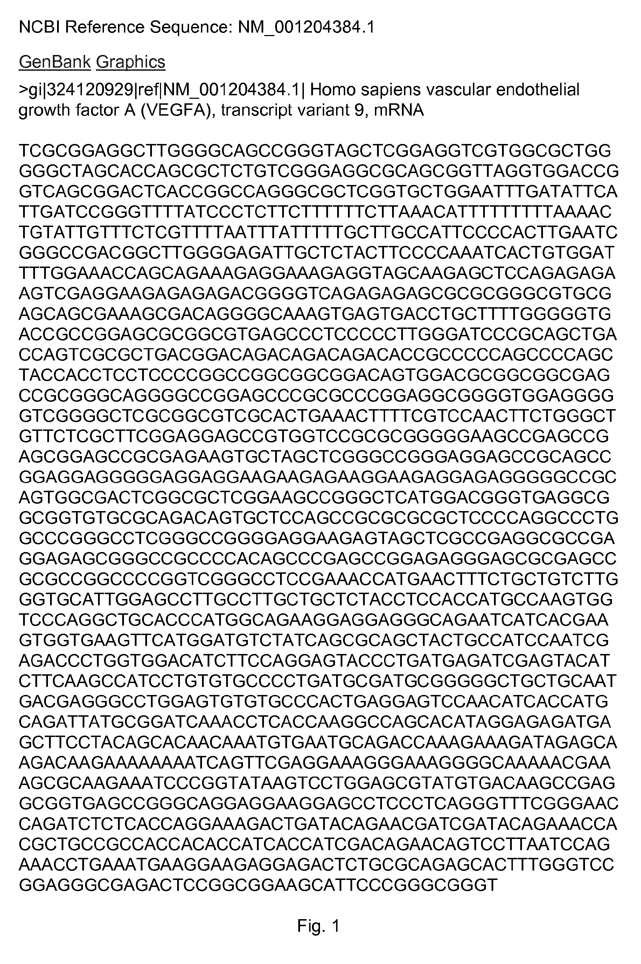

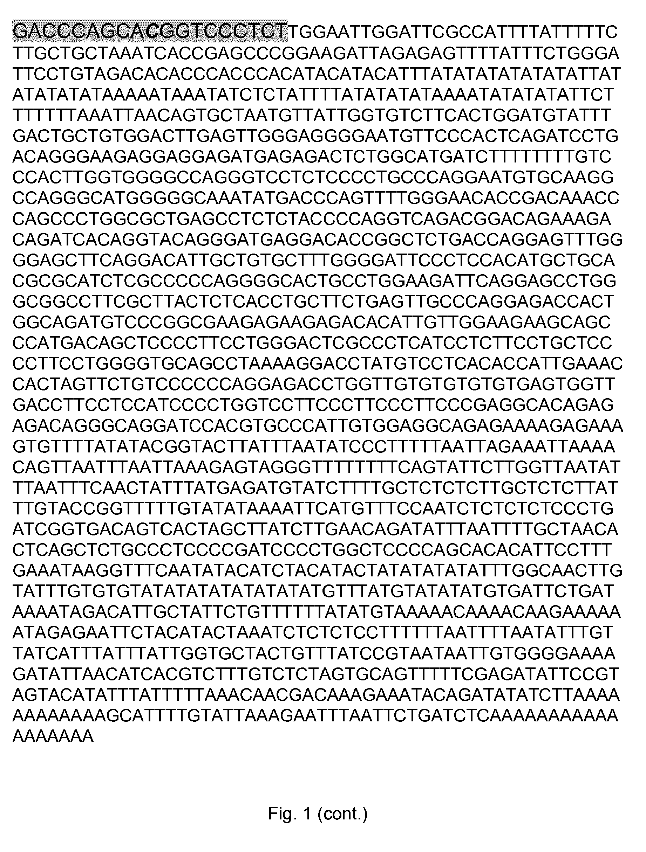

FIG.1. The sequence of the messanger RNA (mRNA) of the human vascular endothelial growth factor A (VEGF-A) is listed in FASTA format. The sequence where the polymorphism is found is marked in a grey box, and highlighted in a bigger font. The crucial polymorphic position +936 (C - cytosine) is depicted in bold and italics right in the middle of the grey box.

### DETAILED DESCRIPTION OF THE INVENTION

The term "polymorphism" as used herein indicates the existance of two or more alleles of a gene which are present in a population with a frequency of at least 1%. Because humans are diploid (that is, there are two sets of chromosomes in each cell), a certain polymorphism can be found in one or both chromosomes which carry the same gene. This means, in the case of the VEGF polymorphism that is used by the invention (which is a change from C to T at position +936), that the change can happen in none, one or two chromosomes. This leads to there being 3 different scenarios: the patient can either have a CC genotype (meaning there is a cytosine [C] at position +936 of the VEGF gene in each of the two chromosomes bearing the VEGF gene), a CT genotype (meaning there is a cytosine [C] at position +936 of the VEGF gene in one chromosome, but a thymine [T] at position +936 of the VEGF gene in the second chromosome), or a TT genotype (meaning there is a thymine [T] at position +936 of the VEGF gene in each of the two chromosomes bearing the VEGF gene). In the present invention, the terms "polymorphism", "single nucleotide polymorphism", and "SNP" are used interchangeably.

The term "acute kidney injury" (AKI) is generally defined as an "abrupt and sustained decrease in kidney function". Because the threshold of separation between normal and impaired kidney function can vary, many different definitions of AKI have been used in the art. The definition used herein follows the standard definition accepted by the RIFLE criteria (Ostermann M., et.al. "The RIFLE criteria" Crit. Care Med. 2007, vol. 35, pp. 2669-2670). It is worthy of mention that AKI, contrary to other conditions affecting the kidney such as chronic kidney disease (CKD) or end stage renal disease (ESRD), is reversible, that is, patients suffering from it can eventually recover and reestablish normal kidney function.

The term "method to determine the risk to develop" as used herein, means a method that is performed in order to find out whether the patient has a genetic predisposition (i.e. he/she is susceptible) to develop the disease.

The term "medical regimen" as used herein, means that a protocol to treat the patient is implemented, in order to improve or maintain said patient's health. This regimen might comprise farmacological treatment and/or surgery.

The term "sepsis" as used herein, describes the medical condition characterized by a whole-body inflammatory state, also called systemic inflammatory response syndrome (SIRS). It is triggered by an infection and usually leads to the presence of organ dysfunction.

The term "septic shock" as used herein, describes a medical condition that is defined as sepsis plus end-organ dysfunction and refractory arterial hypotension.

Vascular endothelial growth factor (VEGF) is a signalling protein that stimulates vasculogenesis and angiogenesis, has chemotactic properties on certain cell types such as macrophages and granulocytes, and is mainly involved in restoring oxygen supply to tissues by the creation of new blood vessels. The human VEGF family has several members called A, B, C, D, E and placenta growth factor. The best known molecule is VEGF-A (referred here as VEGF).The VEGF gene is located on chromosome 6, band p21.3, and comprises eight exons which produce 25 alternative splicing variants and 19 different protein isoforms (VEGF-A - Uniprot entry number P15692 - according to September release 2012). The sequence of the mRNA of the VEGF-A splice variant is found in FIG. 1, together with its NCBI reference number. The C/T polymorphism has been highlighted inside a box.

As it has been described above, a first aspect of the present invention is an *in vitro* method to determine the risk to develop acute kidney injury in a subject, the method comprising the step of detecting the presence of a CC polymorphism at position +936 of the human vascular endothelial growth factor (VEGF) from an isolated sample of the subject, wherein the presence of said polymorphism indicates the risk to develop acute kidney injury

In one particular embodiment of the invention, the *in vitro* method is carried out on an isolated sample of a subject who has been diagnosed of sepsis or septic shock. As is evident from the examples found below, the 936 CC polymorphism of VEGF has been found to associate with the risk to develop AKI in patients suffering from sepsis or septic shock.

In another particular embodiment of the invention, the method is carried out with an isolated sample selected from the group consisting of plasma, blood serum, whole blood, saliva, and urine.

In another particular embodiment of the invention, the isolated sample analyzed in the detection step is a DNA sample.

In another particular embodiment of the invention, the detection of the presence of the CC polymorphism comprises an amplification reaction of the isolated sample.

In another particular embodiment of the invention, the detection of the presence of the CC polymorphism comprises an amplification reaction of the isolated sample using specific oligonucleotides for the vascular endothelial growth factor (VEGF) gene.

In another particular embodiment of the invention, the detection step comprises a hybridization step, a real time polymerase chain reaction (RT-PCR), a Sanger sequencing step, a pyrosequencing step or a combination thereof.

In the present invention, the term "combination thereof" when referred to the above particular embodiment of the first aspect of the invention, means that the detection step comprises one, two, three or all four techniques. Thus, the detection step can comprise a single step selected from: a hybridization step, a real time polymerase chain reaction step, a Sanger sequencing step, and a pyrosequencing step; or the detection step can comprise: hybridization step+RT-PCR, or hybridization step+pyrosenquencing step, or hybrydation step+Sanger sequencing step, or RT-PCR+ pyrosenquencing step, or RT-PCR step+Sanger sequencing step, or Sanger sequencing step+pyrosequencing step; or the detection step can comprise hybridization step+RT-PCR+Sanger sequencing step or hybridization step+RT-PCR+pyrosequencing step, or RT-PCR+Sanger sequencing step+pyrosequencing step, or hybridization step+Sanger sequencing step+pyrosequencing step; or the detection step comprises: hybridization step, a real time polymerase chain reaction (RT-PCR), a Sanger sequencing step, and a pyrosequencing step.

As it has been described above, a third aspect of the invention is the use of means for detecting the presence of the CC polymorphism at position +936 of the human vascular endothelial growth factor (VEGF) from an isolated sample in the method of the first aspect of the invention.

In a particular embodiment of the third aspect, the means used to carry out the invention comprise a biosensor. Biosensors can include a recognizing element, such as a specific antibody or fragment thereof, a probe, an enzyme (bioenzymatic sensor) able to carry out a chemical reaction, and/or a chemical reagents that allow for easy detection of the polymorphism. Once recognition or interaction has taken place, the specific signal of this interaction is detected and translated for easy readout. Examples of signals include electric signals (voltages), optical signals (colour, turbidity) and magnetic fields, among others. Indeed, the technology lying behind biosensors is usually a combination of several disciplines, such as immunology, biochemistry, molecular biology, physics, electronics, optics, nanotechnology, etc.

In yet another particular embodiment of the third aspect, the means used to carry out the invention form part of a kit. In another particular embodiment the kit comprises at least one reagent for the detection of the polymorphism. The reagent for the detection of the polymorphism, which is comprised in the kit, can be a nucleic acid such as DNA or RNA, with a sequence that allows to hybridize specifically with the nucleotide sequence comprising the + 936 polymorphism of VEGF or a pair of primers for hte amplification of the region where the +936 polymorphism is found.

Therefore, in another embodiment, the means for detecting the presence of the polymorphism comprise a biosensor which can form part of a kit with all the components needed to perform the detection reaction (reagents, additives, solvents, etc.) gathered together. Alternatively, the biosensor and other components for performing the analysis may be integers of different kits.

Independently of the means used for the detection of the polymorphism in the *in vitro* method of the invention, it is a preferred embodiment the use of means allowing a point of care testing (POCT). A POCT is defined as medical testing at or near the site of patient care to bring the result of the *in vitro* method of diagnosis conveniently and immediately to the patient. This increases the likelihood that the patient, physician, and care team will receive the results quicker, which allows for immediate clinical management decisions to be made. POCT is often accomplished through the use of transportable, portable, and handheld instruments and test kits.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### EXAMPLES

In order to find out whether some polymorphisms of a group of protein markers were associated to an increased risk of acute kidney injury (AKI) in patients with severe sepsis or septic shock, an observational study was conducted with subjects admitted to the Intensive Care Unit. The association of several polymorphisims to the development of AKI was analyzed by univariate and multivariate multiple logistic regression.

### Methods

### Study design:

A prospective, observational, cohort study was undertaken, screening all patients admitted to the Intensive Care Unit (ICU) of the Hospital Universitario de Getafe from July 2005 to July 2008 with the diagnosis of severe sepsis.

Inclusion criteria were: age over 18 years, diagnosis of severe sepsis on ICU admission and expected ICU length of stay >48 hours.

Exclusion criteria were: second admission to the ICU; history of chronic renal disease defined as a baseline serum creatinine ≥1.40 mg/dl; treatment with chronic dialysis before ICU admission; and absence of informed consent.

### Data collection and definitions:

Inventors collected the results of admission and daily (closest to 8:00 am) arterial blood gases, serum creatinine, glucose and bilirubin concentration, blood platelet count and fluid input, as well as the requirement of renal replacement therapy (RRT) and invasive mechanical ventilation. Information was recorded daily until death, ICU discharge or day 28, whichever came first. Day 0 was the time from ICU admission to 8:00 am on the first ICU day. Day 1 started at 8:00 a.m. on the first ICU day. For each patient inventors calculated the Simplified Acute Physiology Score II (SAPSII) using the worst values within the day 0. Registered chronic comorbidities were also recorded.

Baseline serum creatinine concentration values were estimated using the Modification of Diet in Renal Disease (MDRD) equation, as in previous studies, following the Acute Dialysis Quality Initiative (ADQI) Working Group recommendations, assuming a lower limit of the normal baseline glomerular filtration rate of 75 ml/min (Ostermann M, et.al. "Acute kidney injury in the intensive care unit according to RIFLE" Crit. Care Med. 2007, vol. 35, pp. 1837-43). Fluid input included all fluids infused by intravenous or enteral routes during day 0.

Patients were assigned to the worst RIFLE category according to either peak serum creatinine concentration (the maximum value over a given period of time) or the urine output criteria. AKI was diagnosed in patients who were on the risk, injury or failure RIFLE category during their ICU stay (loss and end stage categories, which require longer than the 28 day follow up, were included in the failure category) (see Ostermann M., mentioned above). Patients could develop AKI during their ICU stay or present to the ICU already with criteria for the diagnosis of AKI. Severe sepsis and septic shock were defined according to the American College of Chest Physicians/Society of Critical Care Medicine (Bernard GR, et. al. "The American-European Consensus Conference on ARDS. Definitions, mechanisms, relevant outcomes, and clinical trial coordination" Am J Respir Crit Care Med. 1994, vol. 149, pp. 818-24). Patient management followed current guidelines (Dellinger RP, et al. "Surviving Sepsis Campaign: international guidelines for management of severe sepsis and septic shock: 2008" Intensive Care Med. 2008, vol. 34, pp. 17-60). The indication for RRT followed standard recommendations (Murray P, et.al. "Renal replacement therapy for acute renal failure" Am J Respir Crit Care Med 2000, vol. 162, pp. 777-81).

### DNA genotyping:

Genomic DNA was extracted from whole blood drawn and isolated according to QIAmp DNA Blood (QIAGEN) kit instruction. Samples were stored at -80°C until use. DNA concentrations were measured with the NanoDrop ND-1 000 Spectrophotometer (NanoDrop Technologies Inc, Wilmington, DE). Angiotensin Converting Enzyme (ACE) Genotyping: ACE insertion/deletion (I/D) polymorphism (rs 4646994) was determined by polymerase chain reaction amplification method as previously described (Rigat B, et.al. "An insertion/deletion polymorphism in the angiotensin I-converting enzyme gene accounting for half the variance of serum enzyme levels" J Clin Invest. 1990, vol. 86, pp. 1343-1346). Special to this assay is the requirement of 3 different PCR primers to obtain the 2 types of allele-specific PCR products. This method yields amplification products of 84 bp for the D allele and 65 pb for the I allele and products were visualized on agarose gels. Single nucleotide polymorphism (SNP) of tumor necrosis factor (TNF) α -376 (rs 1800750), -308 (rs 1800629) and -238 (rs 361525), interleukin (IL)-8 -251 (rs 4073), vascular endothelial growth factor (VEGF) +405 (rs 2010963) and + 936 (rs 3025039) and pre-B-cell colony enhancing factor (PBEF) -1001 (rs9770242) were identified using TaqMan® SNP genotyping assay (for TNFα: C_7514878_10; C_7514879_10 and C_2215707_10; for IL-8: C_11748116_10; for VEGF: C_8311614_10 and C_16198794_10; and for PBEF: C_30014960_10) and Applied Biosystems 7500 Fast Real-time PCR systems.

Physicians were blinded to the genotype results throughout the study period.

### Statistical Analysis:

Deviations from the Hardy-Weinberg equilibrium (HWE) were tested with the public domain program HWE by Rodríguez-Santiago (http://www.oege.org/software/hwe-mr-calc.shtml).

As the allele of risk for each polymorphism is unknown, inventors compared: (a) homozygous wild type *versus* homozygous polymorphic and heterozygous; and (b) homozygous polymorphic *versus* homozygous wild type and heterozygous. The comparison (a or b) that better discriminated the outcome of interest (i.e., the development of AKI) was used to identify the allele of risk and this allele was used for further analysis.

Outcome variables were compared between patients with and without AKI in univariate analysis. Estimative multivariate logistic regression analysis was performed following common rules to develop a model that estimates AKI as the dependent variable, including in the maximal model the variables that at day 0 were associated on univariate analyses with renal dysfunction AKI (p<0.1). In this model the association of only one variable with the outcome of interest can be tested at each time. As only two SNPs were associated with the development of AKI, inventors tested separately the independent association of each one of the two SNPs with the development of AKI.

As expected, patients with AKI presented more disease severity (i.e., higher SAPS> II score and higher incidence of septic shock) than patients without AKI, potentially confounding the analysis on the relationship between any genetic polymorphism and AKI. This methodological problem was approached not only by adjusting in the multivartiate analysis for these (SAPS II, diagnosis of septic shock) and other variables, but also by doing a separate analysis for patients with septic shock.

Continuous variables are expressed as mean ± standard deviation and compared using the Student's t test for normally distributed variables, or the Mann Whitney test for non-parametric variables. Categorical variables are expressed as proportions and were compared by the Chi square test. The strength of the association is expressed as the odds ratio (OR) and its 95% confidence interval (CI). Two-sided p value less than 0.05 was considered statistically significant.

### Results

During the recruitment period, 182 patients with the diagnosis of severe sepsis were admitted to the ICU, 43 had chronic renal failure and 139 fulfilled the inclusion criteria. Sixty five of the 139 recruited patients (46.7 %) presented AKI (62 within the first 48 hours since ICU admission), including 16 (11.5%) in the risk category, 20 (14.4 %) in the injury category and 29 (20.9 %) in the failure category. RRT was required in 14 (10.1 %) patients. As compared with patients without AKI, patients who developed AKI were older, had a higher prevalence of arterial hypertension, were more often in septic shock, presented a higher SAPS II score, had received a larger amount of intravenous fluids during the day 0, and presented a higher ICU mortality (see Table 1 found below).

| Table 1 Clinical characteristis of patients with and without AKI | | | | | | | |
|---|---|---|---|---|---|---|---|
| | | | **All patients (n= 139)** | **AKI (n= 65)** | **Non AKI (n= 74)** | **F value** | |
| | Age | | 60 5= 175 | 649 = 15 8 | 55 7 = 18 0 | <0 01 | |
| | Male gender | | 74 (53.2) | 35 (53 8) | 39 (52.7) | 0 89 | |
| | ICU length of stay | | 11 = 13 | 11 = 13 | 11 = 13 | 0 37 | |
| | ICU mortality | | 35 (25 2) | 23 (35 4) | 12 (16 2) | 0 01 | |

| | Comorbidity | | | | | | |
|---|---|---|---|---|---|---|---|
| | | Arterial hypertension | 49 (35 3) | 30 (46 2) | 19 (25 7) | 0 01 | |
| | | Diabetes | 21 (15 1) | 11 (16 9) | 10 (13 5) | 0 58 | |
| | | Coronary artery disease | 7 (5 0) | 4 (6 2) | 3 (4.1) | 0 57 | |
| | Use of ACE Inhibitor or ARB | | 26 (18 7) | 15 (23 1) | 11 (14 9) | 0 22 | |

| | ICU admission | | | | | | |
|---|---|---|---|---|---|---|---|
| | SAPS II | | 41 5 = 19 4 | 46.5 = 17.5 | 39 4 = 14 4 | <0 01 | |
| | Invasive mechanical ventilation | | 72 (51 8) | 32 (49 2) | 40 (54 1) | 0 57 | |
| | Septic shock | | 74 (53 2) | 41 (63 1) | 33 (44 6) | 0 03 | |
| | Glycema (mg dl) | | 170 3 = 58 5 | 172 3 = 56 4 | 167 8 = 63 0 | 0 72 | |
| | Plalet count x10¹ µL | | 226 = 171 | 182 = 167 | 243 = 192 | 0 24 | |
| | Fluid mput (ml hour) | | 293 = 254 | 360 = 254 | 247 = 249 | <0 01 | |

| | Source of infection | | | | | | |
|---|---|---|---|---|---|---|---|
| | | Respiratory | 45 (32 4) | 12 (18 5) | 33 (44.6) | 0 02 | |
| | | Abdominal | 55 (39 6) | 23 (43 1) | 27 (36 5) | 0 43 | |
| | | Others | 39 (28 0) | 25 (38 6) | 14 (18 9) | 0 01 | |
| Values are expressed as mean =standard deviation or number(%) | | | | | | | |
| See text for abbreviations | | | | | | | |

The frequencies of alleles of each marker gene are shown (Table 2 found below). All polymorphisms were in Hardy Weinberg equilibrium. Ten percent of the samples were randomly selected to repeat the genotyping. The correlation between the first and second genotyping was 100%.

| Table 2 Allele frequencies of the different genes | | | | | | | | |
|---|---|---|---|---|---|---|---|---|
| | **Polymorphism** | **Allele frequency** | **Allele frequency** | **Genotype frequency (**)** | | | **p value** **(Hardy- Winberg equilibrium)** | |
| | TNFα-376 | G | A | GG | GA | AA | >0.05 | |
| | | (94 0) | (6 0) | 122 (8 7) | 17(12 3) | 0 (0 0) | | |
| | INFα-308 | G | A | GG | GA | AA | >0.05 | |
| | | (87 0) | (13 0) | 104 (74 8) | 33 (23 7) | 2 (1.5) | | |
| | TNFα-238 | G | A | GG | GA | AA | >0 05 | |
| | | (96 0) | (4 0) | 127(914) | 12 (8.6) | 0 (0.0) | | |
| | IL 8 -251 | T | A | TT | AT | AA | >0.05 | |
| | | (57.0) | (43.0) | 44 (31.6) | 71 (51.1) | 24 (17.3) | | |
| | PBEF -1001 | T | G | TT | TG | GG | >0 05 | |
| | | (77.0) | (23.0) | 82 (59.0) | 51(36.7) | 6 (4.3) | | |
| | VEGF +936 | c | G | CC | CT | TT | >0 05 | |
| | | (89.0) | (11.0) | 111 (79.9) | 25 (18.0) | 3 (2.1) | | |
| | VEGF +405 | G | C | GG | CG | CC | >0.05 | |
| | | (620) | (38 0) | 50 (36 0) | 72 (51 8) | 17 (12.2) | | |
| | ACE | D | I | DD | ID | II | >0 05 | |
| | | (59.0) | (42.0) | 50 (36.0) | 64 (46.0) | 25 (18.0) | | |
| (*) Percentage: (**) absolute frequency (percentage). See text for abbrevations. | | | | | | | | |

In univariate analyses (see Table 3 found below) only two marker genotypes, namely VEGF +936 CC and PBEF -1001 GG were more frequent in patients with than in patients without AKI. As all patients with the genotype PBEF - 1001 GG suffered AKI, it was not possible to calculate the strength of the association of this specific SNP and AKI.

| | Table 3. Association between different genotypes and AKI (univariate analysis). | | | | | | |
|---|---|---|---|---|---|---|---|
| | **Polymorphism** | **All patiens n=139** | **AKI n = 65** | **Non AKI n= 74** | **OR 95 % CI** | **P** | |
| | PBEF -1001 GG | 6 (4.3) | 6 (4.3) | 0 (0.0) | (*) | <0.01 | |
| | IL 8 -251 AA | 24 (17.3) | 15 (23.1) | 9 (12.2) | 1.51 (0.88 - 2.59) | 0.09 | |
| | VEGF +936 CC | 111 (79.9) | 57 (87.7) | 54 (73.0) | 2.64 (1.07 - 6.49) | 0.03 | |
| | VEGF +405 CC | 17 (12.2) | 9(13.8) | 8(10.8) | 1.33 (0.48 - 3.70) | 0.58 | |
| | ACE II | 25 (18.0) | 11 (16.9) | 14 (18.9) | 1.15 (0.48-2.74) | 0.76 | |
| | FNFα-376 A alelle | 17 (12.2) | 8 (12.3) | 9 (12.2) | 1.01 (0.37-2.80) | 0.98 | |
| | TNFα -308 A alelle | 35 (25.2) | 15 (23.1) | 20 (27.0) | 0.81 (0.37 - 1.75) | 0.59 | |
| | TNFα- 230 A alelle | 12 (8.6) | 7 (10.8) | 5 (6.8) | 1.67 (0.50 - 5.53) | 0.41 | |
| | (*) Odds ratio not determined, as one group did not contain elements. | | | | | | |
| | See text for abbreviations. | | | | | | |

Multivariate analysis included in the maximal model the variables associated with the development of AKI with a p value <0.1 in the univariate analysis, such us age, SAPS II score, the presence of arterial hypertension, the presence of septic shock on ICU admission, the amount of fluid received and the presence of VEGF +936 CC and the PBEF -1001 GG genotypes (see Table 4). After adjusting for other variables, the PBEF -1001 GG genotype was not associated with AKI (p= 0.35). However, the VEGF +936 CC genotype was significantly associated with AKI (see Table 4 found below).

| | | | | |
|---|---|---|---|---|
| Table 4. Association between the polymorphism VEGF +936 CC and the risk of AKI (multivariate analysis). | | | | |

| | | **OR (95% CI)** | **P** | |
|---|---|---|---|---|
| | SAPS II | 1.06 (1.03 - 1.09) | <0.01 | |
| | Chronic hypertension | 3.15 (139 - 7.15) | 0.01 | |
| | VEGF+936 CC | 3.41 (1.19 - 9.79) | 0.02 | |
| See text for abbreviations. | | | | |

The main conclusion from this study is then, that the CC polymorphism is significantly associated to the development of AKI in patients with severe sepsis and septic shock. Therefore, the prospective determination of the polymorphism CC in a patient population can be used as an idication of the risk to develop AKI in sepsis and septic shock.

### REFERENCES CITED IN THE APPLICATION

Vaidya VS., et. al. "Urinary biomarkers for sensitive and specific detection of acute kidney injury in humans" Clin. Transl. Sci. 2008, vol 1, pp. 200-2008
Jaber, BL. et al., "Cytokine gene promoter polymorphisms and mortality in acute renal failure", Cytokine 2004, vol. 25, pp. 212-219
Frank, A. "BCL2 genetic variants are associated with acute kidney injury in septic shock", Crit. Care Med. 2012, vol. 40, pp. 2116-2123
Lu J.C.T. "Searching for genes that matter in acute kidney injury: A systematic review", Clin. J. Am. Soc. Nephrol. 2009, vol. 4, pp. 1020-1030
Medford, A.R.L. et. al. "Vascular endothelial growth factor gene polymorphism and acute respiratory distress syndrome" Thorax 2005, Vvol. 60, pp. 244-248
Ostermann M, et.al. "Acute kidney injury in the intensive care unit according to RIFLE" Crit. Care Med. 2007, vol. 35, pp. 1837-43
Bernard GR, et. al. "The American-European Consensus Conference on ARDS. Definitions, mechanisms, relevant outcomes, and clinical trial coordination" Am J Respir Crit Care Med. 1994, vol. 149, pp. 818-24
Dellinger RP, et al. "Surviving Sepsis Campaign: international guidelines for management of severe sepsis and septic shock: 2008" Intensive Care Med. 2008, vol. 34, pp. 17-60
Murray P, et.al. "Renal replacement therapy for acute renal failure" Am J Respir Crit Care Med 2000, vol. 162, pp. 777-81
Rigat B, et.al. "An insertion/deletion polymorphism in the angiotensin I-converting enzyme gene accounting for half the variance of serum enzyme levels" J Clin Invest. 1990, vol. 86, pp. 1343-1346

## Claims

1. An *in vitro* method to determine the risk to develop acute kidney injury in a subject, the method comprising the step of detecting the presence of a CC polymorphism at position +936 of the human vascular endothelial growth factor (VEGF) from an isolated sample of the subject, wherein the presence of said polymorphism indicates the risk to develop acute kidney injury.

2. The method according to claim 1, wherein the subject has previously been diagnosed of sepsis or septic shock.

3. The method according to any one of claims 1-2, wherein the isolated sample is selected from the group consisting of plasma, blood serum, whole blood, saliva, and urine.

4. The method according to any one of claims 1-3, wherein the isolated sample analyzed in the detection step is a DNA sample.

5. The method according to any one of claims 1-4, wherein the detection of the presence of the CC polymorphism comprises an amplification reaction of the isolated sample.

6. The method according to claim 5, wherein the detection step comprises a hybridization step, a real time polymerase chain reaction, a Sanger sequencing step, a pyrosequencing step, or a combination thereof.

7. A method of deciding or recommending whether to initiate a medical regimen of a subject with a risk of developing AKI, which method comprises determining, *in vitro,* the presence of CC polymorphism at position +936 of VEGF in an isolated sample following the method as defined in any one of the claims 1-6, wherein:
i) if the subject has the risk of developing AKI, then the initiation of the medical regimen is recommended, and
ii) if the subject has not the risk of developing AKI, the follow-up is performed optionally in consideration of the result of an examination of the patient by a physician.

8. Use of means for detecting the presence of the CC polymorphism at position +936 of the human vascular endothelial growth factor (VEGF) from an isolated sample in the methods of any one of the claims 1 to 7.

9. The use according to claim 8, wherein said means comprise a biosensor.

10. The use according to any one of claims 8-9, wherein said means form part of a kit.

11. Use of a CC polymorphism at position +936 of the human vascular endothelial growth factor (VEGF) as marker for determining the risk of developing of acute kidney injury.
